(19) Europäisches Patentamt
European Patent Office
Office européen des brevets

(11) Publication number: 0 349 786 B1

(12) EUROPEAN PATENT SPECIFICATION

(45) Date of publication of patent specification: **10.02.93** (51) Int. Cl.[5]: **C07D 275/02**

(21) Application number: **89110376.4**

(22) Date of filing: **08.06.89**

# (54) Method of stabilizing 3-isothiazolone solution.

(30) Priority: **08.06.88 JP 139485/88**

(43) Date of publication of application:
**10.01.90 Bulletin 90/02**

(45) Publication of the grant of the patent:
**10.02.93 Bulletin 93/06**

(84) Designated Contracting States:
**DE FR GB IT NL SE**

(56) References cited:
**EP-A- 0 166 611** **EP-A- 0 194 146**
**EP-A- 0 208 488** **EP-A- 0 300 483**
**FR-A- 2 257 587** **US-A- 3 870 795**

(73) Proprietor: **ICHIKAWA GOSEI CHEMICAL CO., LTD.**
**676 Naganumaharamachi**
**Chiba-shi Chiba-ken(JP)**

(72) Inventor: **Igarashi, Yoshio**
**736-1 Sonnou-cho**
**Chiba-shi Chiba-ken(JP)**
Inventor: **Chiku, Yukihiro**
**943-2 Kuriyama**
**Yotsukaido-shi Chiba-ken(JP)**
Inventor: **Suzuki, Tetsuya**
**4-1-14-202 Kurosuna**
**Chiba-shi Chiba-ken(JP)**

(74) Representative: **Kador & Partner**
**Corneliusstrasse 15**
**W-8000 München 5(DE)**

EP 0 349 786 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

BACKGROUND OF THE INVENTION

FIELD OF THE INVENTION

This invention is concerned with a new method of stabilizing 3-isothiazolones employed as biocidal and fungicidal agents by the use of solvents.

DESCRIPTION OF THE PRIOR ART

3-isothiazolones are widely employed as biocidal and fungicidal agents. Preparations containing 3-isothiazolones are added to industrial raw materials, a wide variety of products, oils, fuels, cosmetics, fabrics, etc. In the form in which they are used, they are dissolved in organic solvents, or metal salt complexes are formed with them, 3-isothiazolones being very unstable as such, they are stored in a state in which they are dissolved in solvents, and the solutions are prepared as a useful product. A large number of 3-isothiazolones are known. The known 3-isothiazolones include 2-methyl isothiazolone, 2-cyclohexyl isothiazolone, 2-t-butyl isothiazolone, etc., which contains no halogen atom; and 5-chloro-2-methyl isothiazolone, 5-chloro-2-n-hexyl isothiazolone, etc., which contains halogen atoms. The 3-isothiazolones containing halogen atoms are said to have great antimicrobial activities as a rule. However, when such 3-isothiazolones are dispersed in solvents, stabilization is a problem. Especially when heat is applied, the 3-isothiazolones become unstable, and decomposition proceeds rapidly.

U.S. Patent 3,870,795 discloses that non-aqueous solutions containing about 15 % of 5-chloro-2-methyl-3-isothiazolone/2-methyl-3-isothiazolone (93:7) in dipropylene glycol completely decomposed in 28 days at 50 °C. Further, U.S. Patents 4,241,214 and 4,396,413 describe metal salt complexes of the 3-isothiazolone compounds and their use as effective biocidal agents. Besides, in the literature in the art, J. Org. Chem., 30, 2660-2665 (1965) and Aust. J. Chem., 20, 2729-2736 (1967), W. D. Crow et al. describe the stabilization of 3-isothiazolones. It is known that since 3-isothiazolones readily bring about the cleavage of nucleophilic S-N bonding and decompose, solutions which are not stabilized by metal salts decompose rapidly.

Accordingly, current commercial products containing such 3-isothiazolones are sold as aqueous solutions containing divalent nitrate salts which serve as stabilizers for the 3-isothiazolones which would otherwise decompose during storage. Other than the above-described method in which the employment of neutral metal salts and metal nitrates enables the 3-isothiazolones to be stabilized a method is disclosed in Japanese Unexamined Patent Publication No. 61(1986)-212576 in which the addition of various hydroxy solvents and slight amounts of stabilization salts enables the 3-isothiazolones to be stabilized.

However, when the 3-isothiazolones are formed into emulsions, the ions thereof cause shock resulting in a precipitate which leads to deterioration and is an obstacle in production. Further, the precipitation precludes the use of biocides containing the aforesaid salts, especially in situations where mechanical stirring is not feasible.

On the other hand, in the stabilizing method in which hydroxy solvents are used, the stabilizing effect is insufficient. Also in cases where metal salts are used together with hydroxy solvents, solutions diluted with hydroxy solvents promote the cleavage of thiazolone rings due to the protic properties of water (several tens to hundreds of ppm) which is inevitably mixed with the solvents.

When 3-isothiazolones are used as biocides in oils and fuels, it is desirable that preparations thereof do not contain salts. The presence of salts in the preparations results in ignition deposits which lead to clogging and corrosion of various mechanical components. In emulsion-type cosmetics, it is desirable not to use nitrate salts. The elimination of nitrate salts prevents the possibility of nitrosamine formation. Nitrosamines are suspected carcinogens. Accordingly, it is desirable that preparations of 3-isothiazolones do not contain neutral metal salts or metal nitrates and are very stable.

SUMMARY OF THE INVENTION

In view of the above-described problems, the invention provides a new method of stabilizing 3-isothiazolones, which eliminate the drawbacks of the hitherto known stabilizing methods, enables 3-isothiazolones to remain stable over a long period of time, and exerts no bad influence upon materials on which preparations of the 3-isothiazolones are to be used.

According to the invention, 3-isothiazolones can be stabilized over a long period of time by allowing the 3-isothiazolones of formula (1), in which Y is an unsubstituted or substituted alkyl of 1 to 12 carbon atoms, a

halo substituted alkenyl or alkynyl of 2 to 12 carbon atoms, an unsubstituted or substituted cycloalkyl of 5 to 12 carbon atoms, an unsubstituted or substituted aralkyl, or an unsubstituted or substituted aryl, and R is hydrogen, halo, or a ($C_1$-$C_4$) alkyl, to coexist with one or more compounds selected from a group consisting of compounds of formula (2), in which R and $R_1$ are independently hydrogen, or an alkyl of 1 to 4 carbon atoms, X is $-CH_2OCH_2-$ or $-(CH_2)-$ wherein n is an integer of 0 to 4, and $Z_1$ and $Z_3$ are independently -OR or -OCOR containing an alkyl group R of 1 to 4 carbon atoms, compounds of formula (3), in which R and $R_1$ are independently an alkyl of 1 to 4 carbon atoms, compounds of formula (4), in which R is hydrogen, or an alkyl of 1 to 4 carbon atoms, and $R_1$ is an alkyl of 1 to 4 carbon atoms, and compounds of formula (5), in which R is hydrogen, or an alkyl of 1 to 3 carbon atoms.

Formula (1)

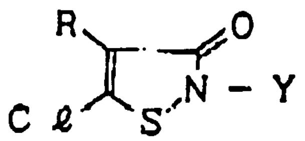

Formula (2)

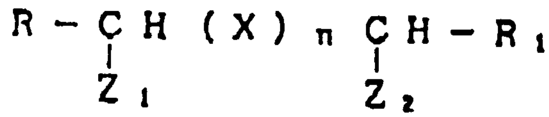

Formula (3)

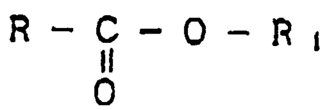

Formula (4)

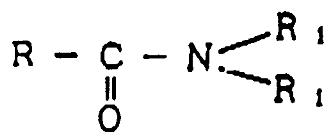

Formula (5)

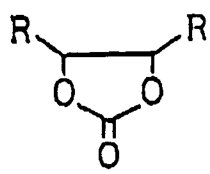

DETAILED DESCRIPTION OF THE INVENTION

Examples of the representative Y substituent of the 3-isothiazolone of formula (1) include methyl, ethyl, propyl, isopropyl, butyl, hexyl, octyl, cyclohexyl, benzyl, 3,4-dichlorobenzyl, 4-methoxybenzyl, 4-chlorobenzyl, 3,4-dichlorophenyl, 4-methoxyphenyl, hydroxymethyl, chloromethyl, chlorophenyl, etc. Examples of the representative 3-isothiazolone include 5-chloro-2-methyl isothiazolone, 5-chloro-2-ethyl isothiazolone, 5-chloro-2-n-hexyl isothiazolone, 4,5-dichloro-2-cyclohexyl isothiazolone, 5-chloro-2-n-octyl isothiazolone, 5-chloro-2-t-octyl isothiazolone, etc. All these 3-isothiazolones have biocidal and fungicidal properties and are used industrially. The methods of manufacturing them are disclosed in Japanese unexamined Patent Publication Nos. 46(1971)-12723 and 45(1970)-38528.

The inventor has made zealous studies of solvents used in the stabilization of 3-isothiazolones in order to determine which preparations of the 3-isothiazolones do not exert a bad influence, such as precipitation, on the materials subjected thereto. As a result he has discovered that the object can be attained by the use of certain solvents. The compounds are represented by the above formulas (2), (3), (4) and (5). Examples of compounds according to formula (2) include acetates, propionates or butyrates of dimethyl ethers, diethyl ethers, dipropyl ethers, dibutyl ethers, monomethyl ethers, monoethyl ethers, monopropyl ethers or monobutyl ethers such as ethylene glycol, propylene glycol, 1,3-propanediol, 1,2-butanediol, 1,3-butanediol, 1,4-butanediol, 2,3-butanediol, 2,4-pentanediol, 1,5 pentanediol, 2,5-hexanediol, 1,6-hexanediol, diethylene glycol, dipropylene glycol, etc.; or diacetates, dipropionates or dibutyrates of glycols. Preferred compounds include ethylene glycol diacetate, ethylene glycol monoethyl ether acetate, ethylene glycol dimethyl ether, ethylene glycol diethyl ether, propylene glycol diacetate, diethylene glycol diacetate, etc.

Examples of compounds according to formula (3) include methyl acetate, ethyl acetate, propyl acetate, butyl acetate, methyl propionate, ethyl propionate, propyl propionate, butyl propionate, methyl butyrate, ethyl butyrate, propyl butyrate, butyl butyrate, methyl valerate, ethyl valerate, propyl valerate, butyl valerate, etc. In particular, ethyl acetate should preferably be used.

Examples of compounds according to formula (4) include dimethylformamide, diethylformamide, dipropylformamide, dibutylformamide, dimethylacetamide, diethylacetamide, dipropylacetamide, dibutylacetamide, dimethylpropionamide, diethylpropionamide, dipropylpropioamide, dibutylpropionamide, etc.

Examples of compounds according to formula (5) include ethylene carbonate, propylene carbonate, 2-oxo-4-ethyl-1,3-dioxolan, 2-oxo-4,5-dimethyl-1,3-dioxolan, etc. Preferred compounds include ethylene carbonate, propylene carbonate, etc.

The compounds represented by formulas (2), (3), (4) and (5) may be used independently or in combination as a solvent for 3-isothiazolone.

The amount of 3-isothiazolone present in a preparation may preferably range from 0.1 to 20 % by weight, and more preferably ranges from 2 to 15 % by weight. A preparation having less than 0.1 % by weight of 3-isothiazolone enhances, the stability of the 3-isothiazolone. However, the antibacterial action of the 3-isothiazolone is relatively weak, and hence the 3-isothiazolone preparation has little value. With more than 20 % by weight, the 3-isothiazolone can hardly be dissolved, and the stability of the 3-isothiazolone deteriorates. The stabilizing solvent is employed in amounts ranging from 80 to 99.9 % by weight, and preferably from 85 to 98 % by weight, on the basis of the amount of 3-isothiazolone used.

Further, 3-isothiazolones remain effective as ingredients of a preparation if they are admixed with, other than the compounds of formula (1), 3-isothiazolones having hydrogen at the chlorine position in formula (1), such as 2-methyl isothiazolone, 2-ethyl isothiazolone, 2-n-butyl isothiazolone, 2-n-hexyl isothiazolone, 2-cyclohexyl isothiazolone, 2-n-octyl isothiazolone, etc., which have relatively excellent stability.

Namely, the stabilizing solvents according to the invention are used for the stabilization of the 3-isothiazolones of formula (1), and may be admixed with 3-isothiazolone having no halogen atom and relatively excellent stability. Further, the stabilizing solvents according to the invention may be used in combination with hydroxy solvents, such as glycols and alcohols, for the purpose of increasing the solubility of 3-isothiazolones, when required. In this case, the amount of stabilizing solvent employed is reduced by the amount of hydroxy solvent used, and the solubility of the 3-isothiazolone increases. However, in view of stability it is desirable to make preparations having proportions falling within the aforesaid range.

Solutions can be prepared by the known method; no special means is required for the preparation. Namely, 3-isothiazolone hydrochloride salt in a reaction solvent obtained through a chlorination cyclization reaction is neutralized with a tertiary organic base or the like, followed by the removal of the precipitated hydrochloride salt of tertiary organic base. Then, the solvent is distilled off from the solution obtained under reduced pressure, and 3-isothiazolone is thereby obtained. A stabilizing solvent is added to the 3-isothiazolone, and a preparation is made therefrom. Solutions of 3-isothiazolones thus obtained possess excellent stability; the 3-isothiazolones act as effective ingredients and do not decompose thermally for a long period of time. The solutions of 3-isothiazolones possess solubilities of several % by weight to several tens of % by weight for water, whereby they can be employed as agueous solutions. The solutions of 3-isothiazolones are also oil soluble; they have excellent affinities for cutting oil, etc. Even if water (several tens to hundred of ppm) is inevitably mingled therewith, the 3-isothiazolones remain stable. Because solutions of the 3-isothiazolones possess the above-described excellent properties, they are useful as watercooling system microbicides, as preservatives for aqueous dispersions or organic polymers, as wood pulp white water slimicides, as cosmetic preservatives, and as cutting oil, jet fuel and heating oil preservatives. Solutions of 3-isothiazolones may also be applied to a solid substrate, such as straw rope, fabric, leather or wood, as a preservative. Solutions of 3-isothiazolones are especially useful as preservatives for oils and fuels, emulsions and dispersions (e.g., paints, floor polishes, binders, etc.) which are sensitive to the addition of salts, and cosmetics, and as watercooling system microbicides.

## EXAMPLES OF THE INVENTION

The following Examples will further illustrate this invention, but are not intended to limit it in any way. All parts and percentages are by weight and all temperatures are given in degrees Centigrade, unless otherwise stated. In evaluating the stability of the products, we assume that one week at a storage temperature of 55 ° C is equivalent to about 3 months at 25 ° C; two weeks is equivalent to about 6 months; four weeks is equivalent to about 12 months, and so on. We consider that any product which does not show signs of decomposition in 4 weeks at 55 ° C (one year at 25 ° C) should be regarded as a stable product.

[Examples]

Examples 1 to 12

Solutions (water content being adjusted to 200 ppm) containing 10 % of 5-chloro-2-methyl isothiazolone and 4 % of 2-methyl isothiazolone dissolved in organic solvents shown in Table 1 were stored at 55 °C, and their stabilities were measured by HPLC. The results which were obtained are shown in Table 1. In all the Examples, 3-isothiazolones did not decompose and the solutions were stable for more than 4 weeks. In Examples other than Examples 7, 8 and 12, the solutions were stable even at 8 weeks.

Table 1

| | Solvent | 2 weeks | 3 weeks | 4 weeks | 6 weeks | 8 weeks |
|---|---|---|---|---|---|---|
| Comparative Example 1 | Ethylene glycol | S | F | F | F | F |
| Comparative Example 2 | Dipropylene glycol | S | F | F | F | F |
| Comparative Example 3 | Polypropylene glycol (MW = 2000) | S | S | F | F | F |
| Comparative Example 4 | Propylene glycol | S | S | F | F | F |
| Example 1 | Ethylene glycol diacetate | S | S | S | S | S |
| Example 2 | Ethylene glycol monoethyl ether acetate | S | S | S | S | S |
| Example 3 | Ethylene glycol dimethyl ether | S | S | S | S | S |
| Example 4 | Ethylene glycol diethyl ether | S | S | S | S | S |
| Example 5 | Propylene glycol diacetate | S | S | S | S | S |
| Example 6 | Diethylene glycol diacetate | S | S | S | S | S |
| Example 7 | Diethylene glycol monoethyl ether acetate | S | S | S | F | F |
| Example 8 | Diethylene glycol dimethyl ether | S | S | S | F | F |
| Example 9 | Ethylene carbonate | S | S | S | S | S |
| Example 10 | Propylene carbonate | S | S | S | S | S |
| Example 11 | Ethyl acetate | S | S | S | S | S |
| Example 12 | Dimethylformamide | S | S | S | F | F |

Note 1)
AI (Active Ingredient) determined by HPLC (Pressure Liquid Chromatography). Each solution was used as a sample to be measured after adjustment of AI concentration to about 14 % by weight.
S: essentially no loss of AI
F: AI totally decomposed

Examples 13 to 16

Solutions containing 10 % of 5-chloro-2-methyl isothiazolone and 4 % of 2-methyl isothiazolone dissolved in organic solvent shown in Table 2 were stored at 55 °C with varied amounts of water contained in the solutions, and the stability of the solutions was measured by HPLC in a similar manner to that of Examples 1 to 12. The results which were obtained are shown in Table 2. In all the Examples. 3-isothiazolones did not decompose for more than 4 weeks. In Examples 13, 14 and 15, the solutions were stable even at 8 weeks, and the solutions remained stable, even in the presence of water which inevitably got mixed with the solutions, not to mention under nonaqueous conditions.

Table 2

| | Solvent (water) | | 2 weeks | 3 weeks | 4 weeks | 6 weeks | 8 weeks |
|---|---|---|---|---|---|---|---|
| Comparative Example 5 | Ethylene glycol | ( 300ppm) | S | S | F | F | F |
| | | ( 700ppm) | S | S | F | F | F |
| | | (1000ppm) | S | F | F | F | F |
| Comparative Example 6 | Propylene glycol | ( 300ppm) | S | S | F | F | F |
| | | ( 700ppm) | S | S | F | F | F |
| | | (1000ppm) | S | F | F | F | F |
| Example 13 | Ethylene glycol diacetate | ( 300ppm) | S | S | S | S | S |
| | | ( 700ppm) | S | S | S | S | S |
| | | (1000ppm) | S | S | S | S | S |
| Example 14 | Ethylene glycol monoethyl ether acetate | ( 300ppm) | S | S | S | S | S |
| | | ( 700ppm) | S | S | S | S | S |
| | | (1000ppm) | S | S | S | S | S |
| Example 15 | Propylene carbonate | ( 300ppm) | S | S | S | S | S |
| | | ( 700ppm) | S | S | S | S | S |
| | | (1000ppm) | S | S | S | S | S |
| Example 16 | Dimethylformamide | ( 300ppm) | S | S | S | S | F |
| | | ( 700ppm) | S | S | S | F | F |
| | | (1000ppm) | S | S | S | F | F |
| Note 1) Water content in the solutions was measured by Karl Fischer's method. | | | | | | | |

Example 17

The propylene carbonate solution of 10 % 5-chloro-2-methyl isothiazolone and 4 % 2-methyl isothiazolone (hereinafter abbreviated to AI) used in Example 10 was diluted with water (10:90, w/w), and solution A containing 1.4 % AI was thereby obtained. On the other hand, the ethylene glycol monoethyl ether acetate solution of 14 % AI used in Example 2 was likewise diluted with water (10:90, w/w), and thereby solution B containing 1.4 % AI was obtained. Further, as a comparative example, 14 % AI solution was diluted to 10 times the original strength with water, and thereby solution C containing 1.4 % AI, 0.9 % $MgCl_2$ and 1.5 % $Mg(NO_3)_2$ was obtained.

These solutions were added to unstirred emulsions (20 ppm AI formed). After being permitted to stand for several minutes, the resulting solutions were filtered through a 10 mesh screen. When solutions A and B were employed, the formation of gum was not observed. When solution C was employed, however, the formation of gum (>100 mg) was observed. Products prepared using the stabilizing solvents according to the invention were excellent; they had no bad influence, such as precipitation, etc., upon materials subjected to them.

Example 18

A solution stabilized by dissolving 2 % of 5-chloro-2-methyl isothiazolone and 0.8 % of 2-methyl isothiazolone in propylene carbonate was subjected to the stabilization test at 55 °C carried out in Examples 1 to 12. As a result, the respective 3-isothiazolones did not decompose, and the solution was

very stable even after 8 weeks. The same solution was added as a preservative to heating oil, so that the amount of 3-isothiazolone became 20 ppm. Since the solution did not contain water, aqueous salts, nitrates, etc., it was stable and could be stored for a long period of time.

Examples 19, 20 and 21

5-chloro-2-(n-octyl) isothiazolone was dissolved in organic solvents so that the content became 10 % by weight. The organic solvents used were ethylene glycol monoethyl ether acetate in Example 19, propylene carbonate in Example 20, and ethylene glycol diacetate in Example 21. The resulting solutions were stored at 55 °C, and the contents of the effective ingredients were followed by HPLC. The results which were obtained are shown in Table 3.

Table 3

| | Solvent(water) | 1 day | 3 days | 8 days | 16 days | 28 days |
|---|---|---|---|---|---|---|
| Comparative Example 7 | Ethylene glycol monoethyl ether | S | S | F | F | F |
| Example 19 | Ethylene glycol monoethyl ether acetate | S | S | S | S | S |
| Example 20 | Propylene carbonate | S | S | S | S | S |
| Example 21 | Ethylene glycol diacetate | S | S | S | S | S |
| Note<br>S: essentially no loss of AI<br>F: AI totally decomposed | | | | | | |

Incidentally, the amount of water was adjusted to 200 ppm.

As a result, it was found that 3-isothiazolone compounds, which have very poor stability in polar solvents, can be stabilized with the method of the invention.

Test Example 1 - Solubility of 5-chloro-2-methyl isothiazolone to the Stabilizing Solvents of the Invention

The solubility of 5-chloro-2-methyl isothiazolone versus that of the stabilizing solvents of the invention was tested for a temperature range of about 0 °C to 25 °C. Solubility curves are shown in Fig. 1.

As a result, it was found that all the solvents subjected to the test have a dissolution potential of about 20 % by weight at around room temperature.

Test Example 2 - Moisture Absorption Test on Solvents

The respective solutions in the Examples 1, 2 and 10 and Comparative Examples 1, 2 and 4 were permitted to stand at room temperature in a room (humidity: 60 to 70 %), under open conditions, and the amount of water which inevitably got mixed therewith due to moisture absorption was followed using a Karl Fischer's moisture meter. The results which were obtained are shown in Fig. 2.

As a result, it was found that the solutions absorb mixture, and the amount of water which gets mixed therewith increases, with the lapse of time. Further, it is to be expected that water will inevitably get mixed with the solutions due to the opening of storage vessels during actual handling.

Examples 22 to 27

5-chloro-2-methyl isothiazolone was dissolved in organic solvents as shown in Table 4 so that the content became 20 % by weight. The resulting solutions were stored at 55 °C, and the contents of the effective ingredients were followed by HPLC. The results which were obtained are shown in Table 4.

As shown in Table 4, the effect of stabilization was clearly observed in examples 22 to 27 as compared with the solution of the comparative examples 8 to 10, through the stability of the solutions of examples 22 to 27 was a little lowered as the temperature rised.

Table 4

| | Solvent | 2 weeks | 3 weeks | 4 weeks | 6 weeks |
|---|---|---|---|---|---|
| Comparative Example 8 | Ethylene glycol | S | F | F | F |
| Comparative Example 9 | Dipropylene glycol | S | F | F | F |
| Comparative Example 10 | Polypropylene glycol (MW=2000) | S | F | F | F |
| Example 22 | Ethylene glycol diacetate | S | S | S | S |
| Example 23 | Ethylene glycol monoethyl ether acetate | S | S | S | S |
| Example 24 | Propylene glycol diacetate | S | S | S | S |
| Example 25 | Propylene carbonate | S | S | S | S |
| Example 26 | Ethyl acetate | S | S | F | F |
| Example 27 | Dimethylformamide | S | S | F | F |

Note 1)

AI (Active Ingredient) determined by HPLC. Each solution was used as a sample to be measured after adjustment of AI concentration to about 20 % by weight.

S: essentially no loss of AI

F: AI totally decomposed

## Claims

1. A method of stabilizing 3-isothiazolones, which comprises the step of providing a solution of a 3-isothiazolones of formula (1), in which Y is an unsubstituted or substituted alkyl of 1 to 12 carbon atoms, a halo substituted alkenyl or alkynyl of 2 to 12 carbon atoms, an unsubstituted or substituted cycloalkyl of 5 to 12 carbon atoms, an unsubstituted or substituted aralkyl, or an unsubstituted or substituted aryl, and R is hydrogen, halo, or a ($C_1$-$C_4$) alkyl, in a solvent comprising one or more compounds selected from a group consisting of compounds of formula (2), in which R and $R_1$ are independently hydrogen, or an alkyl of 1 to 4 carbon atoms, X is - $CH_2OCH_2$- or -($CH_2$)- wherein n is an integer of 0 to 4, and $Z_1$ and $Z_2$ are independently -OR or -OCOR containing an alkyl group R of 1 to 4 carbon atoms, compounds of formula (3), in which R and $R_1$ are independently an alkyl of 1 to 4 carbon atoms, compounds of formula (4), in which R is hydrogen, or an alkyl of 1 to 4 carbon atoms, and $R_1$ is an alkyl of 1 to 4 carbon atoms, and compounds of formula (5), in which R is hydrogen, or an alkyl of 1 to 3 carbon atoms.

Formula (1)

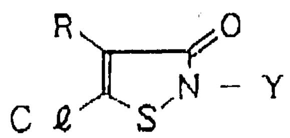

Formula (2)

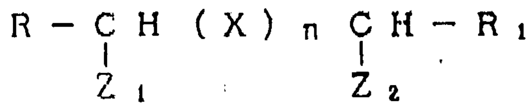

Formula (3)

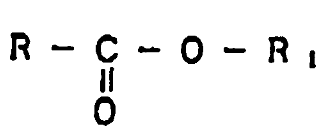

Formula (4)

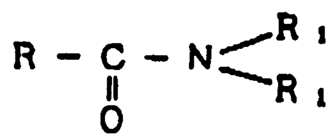

Formula (5)

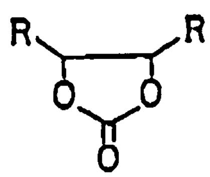

2. A method as claimed in claim 1, wherein the 3-isothiazolone content ranges from 0.1 to 20 % by weight.

3. A method as claimed in claim 1 or 2, wherein the solvent content ranges from 80 to 99.9 % by weight.

4. A method as claimed in claim 1 or 3, wherein the 3-isothiazolone content ranges from 2 to 15 % by weight.

5. A method as claimed in claim 1, 2 or 4, wherein the solvent content ranges from 85 to 98 % by weight.

6. 3-isothiazolone solution stabilized by the method according to claim 1, comprising 3-isothiazolones represented by said formula (1) and at least one of the compounds as a solvent represented by said formulae (2) to (5).

## Patentansprüche

1. Verfahren zur Stabilisierung von 3-Isothiazolonen, das den Schritt der Bereitstellung einer Lösung von 3-Isothiazolonen der Formel (1), worin Y ein unsubstituiertes oder substituiertes Alkyl mit 1 bis 12 Kohlenstoffatomen, ein mit Halogen substituiertes Alkenyl oder Alkinyl mit 2 bis 12 Kohlenstoffatomen, ein unsubstituiertes oder substituiertes Cycloalkyl mit 5 bis 12 Kohlenstoffatomen, ein unsubstituiertes oder substituiertes Aralkyl oder ein unsubstituiertes oder substituiertes Aryl ist, und R Wasserstoff, ein Halogen oder ein ($C_1$-$C_4$)-Alkyl ist, in einem Lösungsmittel umfaßt, das eine oder mehrere Verbindungen umfaßt, die aus der Gruppe ausgewählt sind, die aus Verbindungen der Formel (2), worin R und $R_1$ unabhängig voneinander Wasserstoff oder ein Alkyl mit 1 bis 4 Kohlenstoffatomen sind, X $-CH_2OCH_2-$ oder $-(CH_2)-$ ist, worin n eine ganze Zahl von 0 bis 4 ist und $Z_1$ und $Z_2$ unabhängig voneinander -OR oder -OCOR sind, die eine Alkylgruppe R mit 1 bis 4 Kohlenstoffatomen enthalten, Verbindungen der Formel (3), worin R und $R_1$ unabhängig voneinander ein Alkyl mit 1 bis 4 Kohlenstoffatomen sind, Verbindungen der Formel (4), worin R Wasserstoff oder ein Alkyl mit 1 bis 4 Kohlenstoffatomen ist und

$R_1$ ein Alkyl mit 1 bis 4 Kohlenstoffatomen ist, und Verbindungen der Formel (5) bestehen, worin R Wasserstoff oder ein Alkyl mit 1 bis 3 Kohlenstoffatomen ist.

Formel (1)

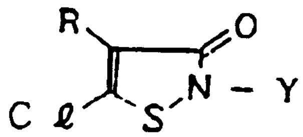

Formel (2)

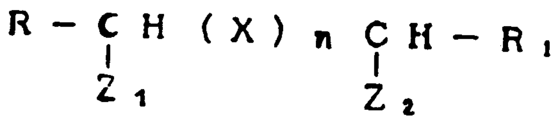

Formel (3)

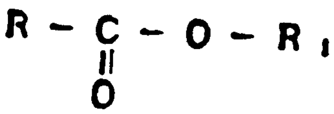

Formel (4)

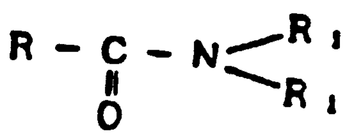

Formel(5)

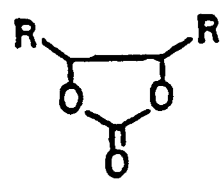

2. Verfahren nach Anspruch 1, worin der 3-Isothiazolongehalt im Bereich von 0,1 bis 20 Gew-% liegt.

3. Verfahren nach Anspruch 1 oder 2, worin der Lösungsmittelgehalt im Bereich von 80 bis 99,9 Gew-% liegt.

4. Verfahren nach Anspruch 1 oder 3, worin der 3-Isothiazolongehalt im Bereich von 2 bis 15 Gew-% liegt.

5. Verfahren nach Anspruch 1, 2 oder 4, worin der Lösungsmittelgehalt im Bereich von 85 bis 98 Gew-% liegt.

6. Nach dem Verfahren von Anspruch 1 stabilisierte 3-Isothiazolonlösung, die 3-Isothiazolone der Formel (1) und zumindest eine der Verbindungen als Lösungsmittel umfaßt, die durch die Formeln (2) bis (5) dargestellt werden.

**Revendications**

1. Procédé de stabilisation de 3-isothiazolones, qui comprend l'étape consistant à mettre une solution d'une 3-isothiazolone de formule (1), dans laquelle Y est un alcoyle non-substitué ou substitué en $C_{1-12}$, un alcényle ou alcynyle halo-substitué en $C_{2-12}$, un cycloalcoyle non-substitué ou substitué en $C_{5-12}$, un aralcoyle non-substitué ou substitué, ou un aryle non-substitué ou substitué, et R est un hydrogène, un halo ou un alcoyle en $C_{1-4}$, dans un solvant comprenant un ou plusieurs composés choisis dans un groupe constitué des composés de formule (2), dans laquelle R et $R_1$ représentent indépendamment un hydrogène, ou un alcoyle en $C_{1-4}$, X représente $-CH_2OCH_2-$ ou $-(CH_2)-$ où n est un nombre entier valant de 0 à 4, et $Z_1$ et $Z_2$ représentent indépendamment -OR ou -OCOR contenant un groupe alcoyle R en $C_{1-4}$, composés de formule (3) dans laquelle R et $R_1$ représentent indépendamment un alcoyle en $C_{1-4}$, composés de formule (4) dans laquelle R est un hydrogène ou un alcoyle en $C_{1-4}$ et $R_1$ est un alcoyle en $C_{1-4}$, et composés de formule (5), dans laquelle R est un hydrogène, ou un alcoyle en $C_{1-3}$.

Formule (1)

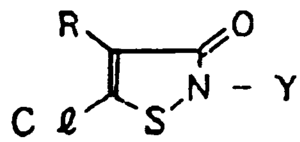

Formule (2)

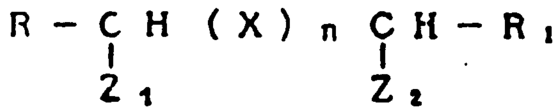

Formule (3)

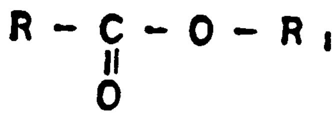

Formule (4)

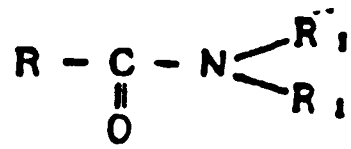

Formule (5)

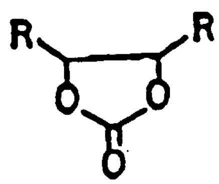

2. Procédé selon la revendication 1, dans lequel la teneur en 3-isothiazolone se situe entre 0,1 et 20% en poids.

3. Procédé selon la revendication 1 ou 2, dans lequel la teneur en solvant se situe entre 80 et 99,9% en poids.

4. Procédé selon la revendication 1 ou 3, dans lequel la teneur en 3-isothiazolone se situe entre 2 et 15% en poids.

5. Procédé selon la revendication 1, 2 ou 4, dans lequel la teneur en solvant se situe entre 85 et 98% en poids.

6. Solution de 3-isothiazolone stabilisée par le procédé selon la revendication 1, comprenant des 3-isothiazolones représentées par ladite formule (1) et au moins un des composés tels qu'un solvants représenté par lesdites formules (2) à (5).

FIG.1

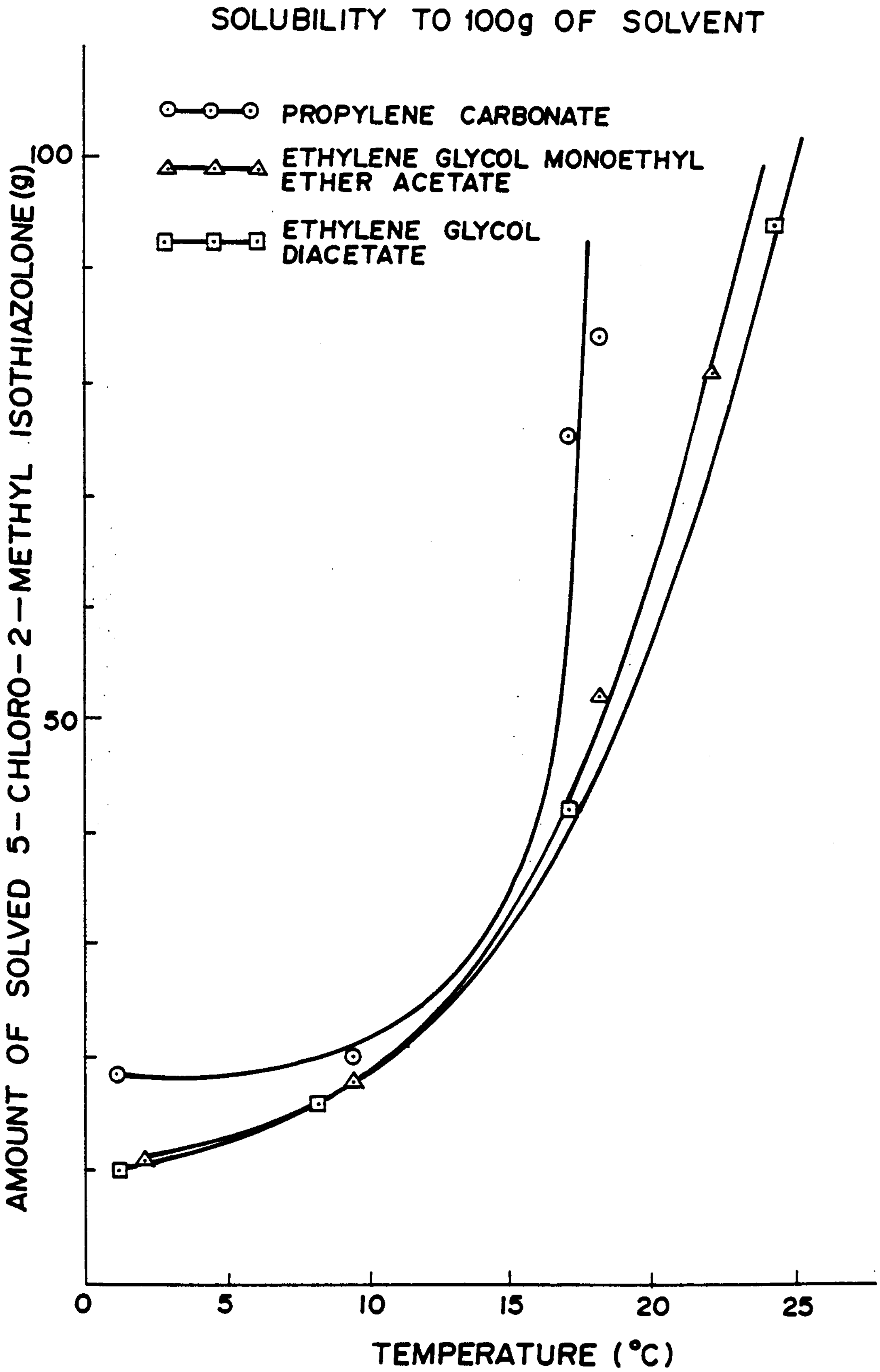
SOLUBILITY TO 100g OF SOLVENT
PROPYLENE CARBONATE
ETHYLENE GLYCOL MONOETHYL ETHER ACETATE
ETHYLENE GLYCOL DIACETATE
AMOUNT OF SOLVED 5-CHLORO-2-METHYL ISOTHIAZOLONE (g)
100
50
0
5
10
15
20
25
TEMPERATURE (°C)

FIG.2

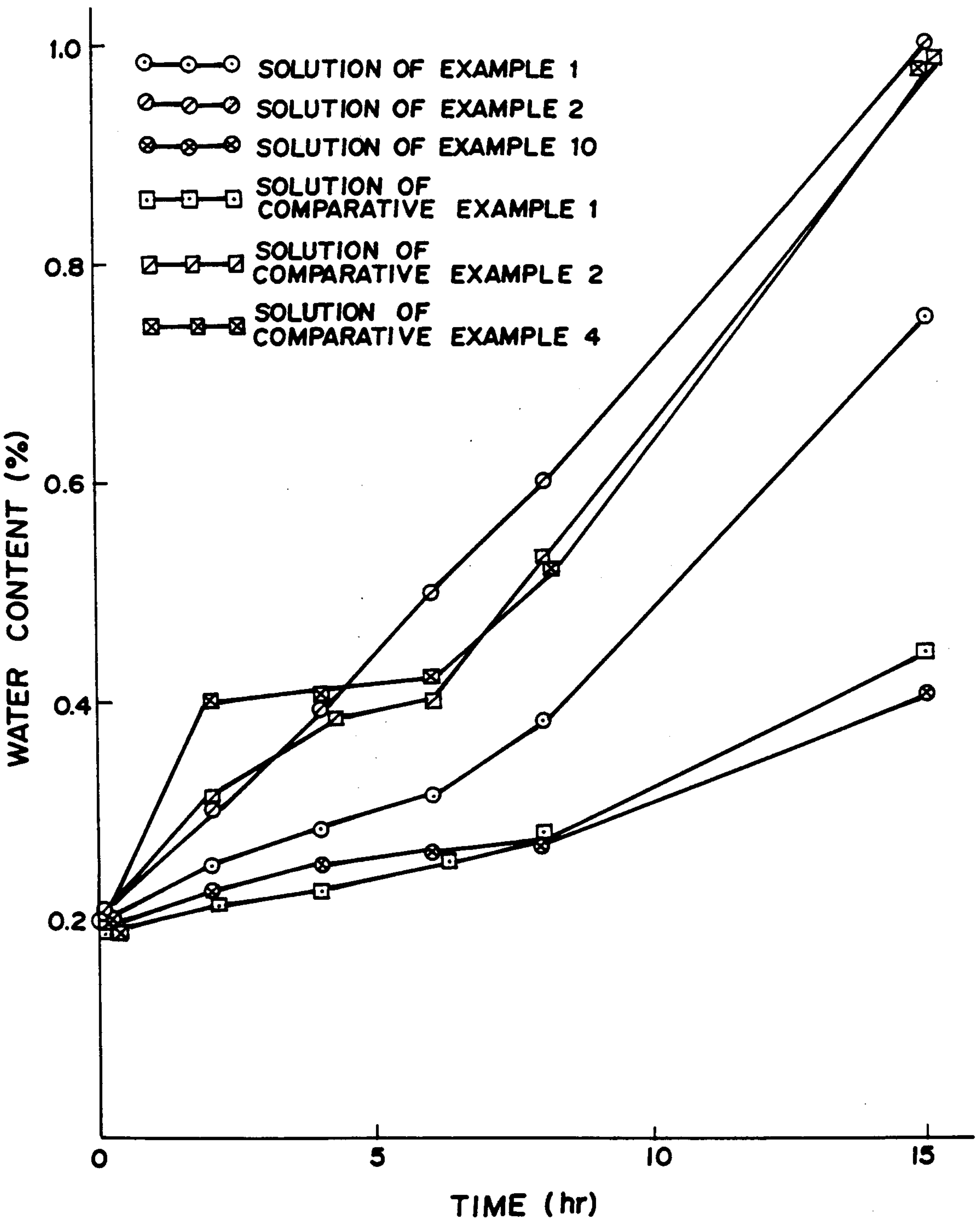
SOLUTION OF EXAMPLE 1
SOLUTION OF EXAMPLE 2
SOLUTION OF EXAMPLE 10
SOLUTION OF COMPARATIVE EXAMPLE 1
SOLUTION OF COMPARATIVE EXAMPLE 2
SOLUTION OF COMPARATIVE EXAMPLE 4
WATER CONTENT (%)
1.0
0.8
0.6
0.4
0.2
0
5
10
15
TIME (hr)